(19) Europäisches Patentamt
European Patent Office
Office européen des brevets

(11) EP 0 950 401 B1

(12) FASCICULE DE BREVET EUROPEEN

(45) Date de publication et mention de la délivrance du brevet:
**09.01.2008 Bulletin 2008/02**

(51) Int Cl.:
***A61K 8/893*** *(2006.01)* ***A61Q 1/00*** *(2006.01)*

(21) Numéro de dépôt: **99400681.5**

(22) Date de dépôt: **19.03.1999**

(54) **Utilisation en cosmétique d'émulsions E/H comprenant une silicone oxyalkylénée substituée en alpha-omega**

Kosmetische Verwendung von W/Ö Emulsionen enthaltend alpha-omega-substituierte oxyalkylierte Silicone

Cosmetic use of W/O emulsions comprising an alpha-omega oxyalkylated substituted silicone

(84) Etats contractants désignés:
**DE ES FR GB IT**

(30) Priorité: **26.03.1998 FR 9803761**

(43) Date de publication de la demande:
**20.10.1999 Bulletin 1999/42**

(73) Titulaire: **L'ORÉAL**
**75008 Paris (FR)**

(72) Inventeurs:
- **Bara, Isabelle**
  **75013 Paris (FR)**
- **Lemann, Patricia**
  **94000 Creteil (FR)**
- **Tounilhac, Florence**
  **75011 Paris (FR)**
- **Collette, Annick**
  **94600 Choisy Le Roi (FR)**
- **Simon, Jean-Christophe**
  **75012 Paris (FR)**

(74) Mandataire: **Boulard, Denis**
**L'OREAL - D.I.P.I.**
**25-29 Quai Aulagnier**
**92600 Asnières (FR)**

(56) Documents cités:
**EP-A- 0 331 833**
**EP-A- 0 373 661**
**EP-A- 0 583 130**
**EP-A- 0 612 517**
**EP-A- 0 796 615**
**EP-A- 0 819 426**

- **DATABASE WPI Week 8644 Derwent Publications Ltd., London, GB; AN 86-288910 XP002090853 "Emulsifier compsns. esp. for treating silicone oil - obtd. by treating organic modified clay mineral polyoxyalkylene modified organo polysiloxane" & JP 61 212321 A (SHISEIDO), 20 septembre 1986 (1986-09-20)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des émulsions eau-dans-huile (E/H), des compositions à usage cosmétique, comprenant une telle émulsion ainsi que leur utilisation dans les domaines cosmétique,

Ces compositions peuvent constituer des produits de soin de la peau, y compris le cuir chevelu, et/ou des produits de maquillage de la peau, des muqueuses (lèvres ou intérieurs des paupières), des semi-muqueuses (lèvres), des fibres kératiniques (cheveux, cils, ongles) ou encore des produits de maquillage du corps.

Les compositions de maquillage, en particulier de fonds de teint se présentent généralement sous forme de crème plus ou moins fluide comprenant des corps gras tels que des huiles et une phase particulaire généralement composée de charges et de pigments.

On cherche en général à introduire dans la phase grasse des composés tels que les silicones qui apportent de la douceur et de la fluidité. Toutefois, il est connu que plus la teneur en huile de silicone augmente, plus l'obtention d'une émulsion E/H stable est difficile non seulement dans le temps mais également lorsqu'elle est soumise à d'importantes variations de température. En effet, la fluidité de la formule peut être à l'origine de phénomènes d'instabilités au cours du temps, tels que relargage d'huile en surface, sédimentation des pigments, épaississement, etc...

Dans le but de diminuer ces phénomènes, il a été proposé dans US 4.698.178 d'utiliser une nouvelle classe de tensioactifs siliconés associés à des polyols pour les températures basses et à des électrolytes ou à des savons métalliques pour les températures élevées.

L'amélioration de la stabilité des émulsions E/H a également été étudiée dans la demande EP 331.833 qui décrit l'utilisation de silicones oxyalkylénées associés à des argiles minérales gonflables à l'eau et dans la demande EP-A-612 517 qui préconise l'utilisation d'une association d'une silicone à groupements oxyalkylène et alkyle pendants avec un agent gélifiant et/ou épaississant.

Néanmoins, ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, un vêtement ou la peau.

En se déposant, ladite composition laisse une trace sur ledit support. Il s'en suit donc une persistance médiocre de la composition sur la peau ou les muqueuses, d'où la nécessité de renouveler régulièrement son application.

Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint ; dans les ridules qui entourent les lèvres, dans le cas des rouges à lèvres ; dans les plis de la paupière, dans le cas des fards à paupières. On a également constaté, dans le cas notamment des fards à paupières, l'apparition de stries dans le maquillage, générées par les mouvements des paupières.

Tous ces phénomènes engendrent un effet inesthétique que l'on souhaite bien évidemment éviter.

Le but de la présente invention est de fournir une émulsion eau-dans-huile qui présente une bonne stabilité, tout en conservant de bonnes propriétés cosmétiques. En particulier, il est recherché de pouvoir disposer d'une émulsion eau-dans-huile stable qui ne transfère pas après son application, notamment sur la peau.

Il a maintenant été découvert de façon inattendue et surprenante que par l'emploi d'une silicone oxyalkylénée particulière, il était possible d'obtenir des émulsions E/H ayant une viscosité particulière, ces émulsions ayant non seulement une bonne stabilité dans le temps mais encore vis-à-vis des variations de température et présentant de plus d'excellentes propriétés cosmétiques ainsi qu'une bonne résistance au transfert.

La présente invention a donc pour objet l'utilisation d'une émulsion eau-dans-huile de maquillage, comprenant une phase aqueuse et une phase grasse comprenant une huile de silicone, caractérisée par le fait qu'elle comprend une phase particulaire comprenant des pigments et/ou des nacres et/ou des charges, et au moins une silicone oxyalkylénée substituée en $\alpha$-$\omega$ aux deux extrémités de la chaîne principale, la composition ayant une viscosité dynamique allant de 100 mPa.s à 20 Pa.s, cette viscosité étant mesurée à 25 °C à un taux de cisaillement de 200 $s^{-1}$ pour obtenir un maquillage qui ne transfère pas après son application sur la peau.

L'invention porte également sur un procédé de traitement non thérapeutique de la peau et/ou des fibres kératiniques, notamment un procédé de maquillage, consistant à appliquer sur la peau et/ou sur les fibres kératiniques une émulsion et/ou une composition telles que définies ci-dessus.

L'émulsion E/H selon l'invention répond parfaitement aux normes de stabilité soit :

- résistance à l'épreuve de centrifugation à 4000 tr/mn pendant 1 heure,
- résistance au vieillissement à température ambiante (25 °C) pendant 2 mois à température ambiante (25 °C) et ainsi qu'à 45 °C,
- résistance à 8 cycles successifs de 8 heures chacun dont les températures s'échelonnent de -20 °C à +20 °C.

L'émulsion selon l'invention répond aux critères suivants :

- elle a et conserve au cours de ces tests un aspect macroscopique et microscopique homogène et stable (globules finement dispersés, absence de relargage) et
- sa viscosité est constante au cours du temps.

L'émulsion selon l'invention présente également une très bonne résistance au transfert. De plus, l'émulsion appliquée sur la peau présente l'avantage de ne pas migrer dans les plis de la peau en particulier sur les paupières et/ou les rides du visage, en particulier au niveau des lèvres et des yeux, et de leurs contours (patte d'oie).

On a constaté que l'émulsion utilisée selon l'invention s'applique et s'étale facilement de façon homogène, sans laisser de sensation de gras et présente de bonnes propriétés cosmétiques. Le film obtenu présente également une texture légère et reste confortable à porter tout au long de la journée.

Par ailleurs, il est possible d'ajouter à l'émulsion selon l'invention d'autres adjuvants tels que des huiles et/ou des poudres (pigments et/ou charges) tout en conservant une émulsion stable. L'émulsion est donc compatible avec un grand nombre d'adjuvants cosmétiques.

L'émulsion selon l'invention possède par ailleurs de bonnes qualités sensorielles notamment une grande facilité d'application, du confort, de la douceur, une bonne matité et une bonne couvrance, de l'uniformité et de la tenue.

L'émulsion selon l'invention comprend une silicone oxyalkylénée substituée en $\alpha$-$\omega$.

Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en $C_1$-$C_{10}$ et en particulier méthyle, les radicaux fluoroalkyles, les radicaux aryles et en particulier phényle. Ils peuvent être par exemple substitués par des groupements ester ou éther en $C_1$-$C_{40}$, des groupements aralkyles en $C_7$-$C_{60}$.

Ainsi, la silicone oxyalkyléne substituée en $\alpha$-$\omega$ utilisable pour le système émulsionnant de l'émulsion selon l'invention est un polymère organosilicié tel que défini ci-dessus, à structure linéaire, substitué aux deux extrémités de la chaîne principale par des groupements oxyalkylène reliés aux atomes de Si par l'intermédiaire d'un groupement hydrocarboné.

De préférence, la silicone oxyalkylénée substituée en $\alpha$-$\omega$ répond à la formule générale (I) suivante :

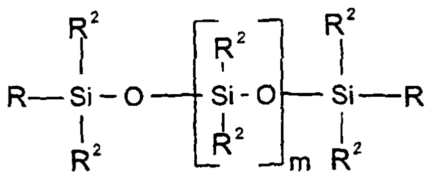

(I)

dans laquelle: R = $-(CH_2)_p\ O-(C_2H_4O)_x\ (C_3H_6O)_Y R^1$
où :- $R^1$ représente H, $CH_3$ ou $CH_2CH_3$,

- p est un entier allant de 1 à 5, x varie de 1 à 100, y varie de 0 à 50,
- les unités ($C_2H_4O$) et ($C_3H_6O$) pouvant être réparties de façon aléatoire ou par blocs,
- les radicaux $R^2$ représentent un radical alkyle en C1-C3 ou un radical phényle,
- $5 \leq m \leq 300$.

De préférence, la silicone oxyalkylénée substituée en $\alpha$-$\omega$ utilisée selon la présente invention répond à la formule générale (I) pour laquelle tous les radicaux $R^2$ sont des radicaux méthyles et :

- p va de 2 à 4,
- x va de 3 à 100,

- m va de 50 à 200.

De préférence encore, le poids moléculaire moyen de R va de 800 à 2600.
De préférence, le rapport en poids des unités $C_2H_4O$ par rapport aux unités $C_3H_6O$ va de 100:10 à 20:80.
De préférence, ce rapport est d'environ 42/58.
De préférence encore, $R^1$ est le groupe méthyle.
De façon plus préférentielle encore, l'émulsion selon l'invention comprend la silicone oxyalkylénée en $\alpha$-$\omega$ de formule suivante :

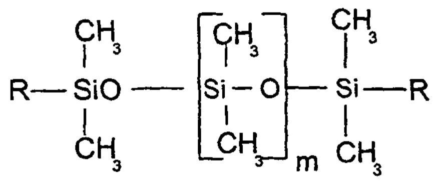

dans laquelle :

- m = 100,
- R = $(CH_2)_3$-O-$(C_2H_4O)_x$-$(C_3H_6O)_y$-$CH_3$, où x va de 3 à 100, y va de 1 à 50, le rapport en poids du nombre de $C_2H_4O$ sur le nombre de $C_3H_6O$ étant d'environ 42/58, le poids moléculaire moyen de R allant de 800 à 1000.

La silicone oxyalkylénée substituée en $\alpha$-$\omega$ telle que définie ci-dessus est utilisée selon l'invention en une proportion allant de 0,1 à 30 % de préférence de 0,5 à 10 % en poids par rapport au poids total de l'émulsion.
Parmi les produits du commerce pouvant contenir tout ou partie des silicones oxyalkylénées substituées en $\alpha$-$\omega$ utilisables selon l'invention comme émulsionnant, on peut citer notamment ceux vendus sous les dénominations de "Abil EM 97" par la Société Goldschmidt, ou encore de "KF 6009", "X22-4350", "X22-4349" ou "KF 6008" par la Société Shin Etsu.
La phase grasse des émulsions selon l'invention comprend au moins une huile de silicone, volatile ou non.
L'huile de silicone utilisable selon l'invention peut être un polydiorganosiloxane linéaire, éventuellement fonctionnalisé, ou cyclique ou un organopolysiloxane éventuellement réticulé ou un mélange de ceux-ci.
Les polydiorganosiloxanes linéaires éventuellement fonctionnalisés utilisables selon l'invention répondent à la formule générale suivante :

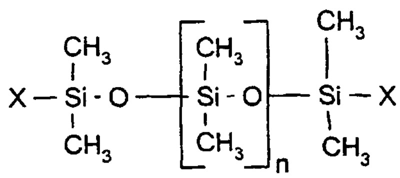

dans laquelle:

- X est $-CH_3$ ou OH, et
- n est un entier allant de 0 à 2000.

Parmi ceux-ci, on citera notamment les produits vendus sous la dénomination de "AK" par la Société WACKER, "SF" par la Société GENERAL ELECTRIC et "ABIL" par la Société GOLDSCHMIDT, tel que le produit "Abil 10".
Comme polydiorganosiloxanes cycliques selon l'invention, on peut utiliser, seuls ou en mélange, des cyclométhicones de formule :

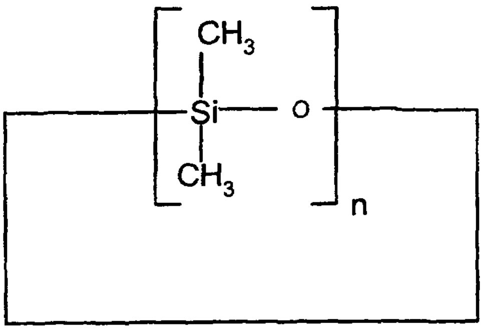

dans laquelle :

- n est un nombre entier de 3 à 8.

Parmi les cyclométhicones particulièrement préférées, on citera le cyclotétradiméthylsiloxane (n = 4), le cyclopentadiméthylsiloxane (n = 5), et le cyclohexadiméthylsiloxane (n = 6).

On peut notamment utiliser les produits vendus sous les dénominations de "DC Fluid 244", "DC Fluid 245", "DC FLuid 344 et "DC Fluid 345" par la Société Dow Corning.

D'autres cyclométhicones utilisables selon l'invention sont celles vendues sous les dénominations de "Abil K4" par la Société GOLDSCHMIDT ; sous les dénominations de "Silbione 70045 V2" et de "Silbione Huile 70045 V5" par la Société RHONE POULENC ; ainsi que sous les dénominations de "Volatil Silicone 7158" et de "Volatil Silicone 7207" par la Société UNION CARBIDE.

De préférence, on utilise les huiles de silicone volatiles, dont les cyclométhicones.

Tel qu'indiqué ci-dessus, l'huile de silicone utilisée selon l'invention est de préférence présente en une proportion d'au moins 5 % et de préférence allant de 25 à 45 % en poids par rapport au poids total de l'émulsion.

Les compositions de l'invention peuvent également comprendre d'autres composés siliconés.

Parmi ces composés siliconés, on peut citer les polyalkyl($C_1$-$C_{20}$)siloxanes, dont les huiles de silicone phénylées, ainsi que les gommes de silicones et les cires de silicone.

Les gommes de silicone utilisables dans la composition de l'invention peuvent être des polysiloxanes de masse moléculaire élevée, de l'ordre de 200 000 à 1 000 000 et de viscosité supérieure à 500 000 mPa.s. Elles peuvent être utilisées seules ou en mélange avec un solvant tel qu'une huile polydiméthylsiloxane ou polyphénylsiloxane, ou une cyclométhicone.

Les gommes peuvent être présentes jusqu'à 5 % en poids de matière active dans la composition finale, de préférence jusqu'à 1 %.

De préférence encore, la composition selon l'invention est substantiellement exempte de gomme de silicone.

Les cires de silicone utilisables dans la composition selon l'invention peuvent être des polysiloxanes linéaires substitués. On peut citer, par exemple, les cires de silicone polyéther, les alkyl ou alkoxy-diméthicones ayant de 16 à 45 atomes de carbone. Ces cires de silicone peuvent être présentes à raison de 0 à 15 % en poids dans la composition finale, de préférence à raison de 2 à 10 %.

Les émulsions selon l'invention peuvent également comprendre des résines de silicone comprenant une combinaison des unités $R_3SiO_{1/2}$, $R_2SiO_{2/2}$, $RSiO_{3/2}$ et $SiO_{4/2}$.

Les compositions selon l'invention peuvent également comprendre des corps gras non siliconés dont des corps gras pâteux, des gommes et des cires d'origine végétale, minérale, animale ou synthétique.

On peut définir les composés gras pâteux à l'aide d'au moins une des propriétés physico-chimiques suivantes :

- une viscosité de 0,1 à 40 Pa.s (1 à 400 poises), mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

Comme cires utilisables dans l'invention, on peut citer les cires d'origine animale comme la lanoline, la cire d'abeilles, le spermaceti, les dérivés de la lanoline tels que les alcools de lanoline, la lanoline hydrogénée, hydroxylée ou acétylée, les acides gras de la lanoline et l'alcool de lanoline acétylée ; les cires d'origine végétale telles que la cire de Carnauba, de Candellila, de kapok, d'Ouricury, de riz, de jojoba hydrogénée, d'Alfa, du Japon ou les cires de fibres

de liège ou de canne à sucre ou encore le beurre de cacao ; les cires minérales par exemple de paraffine, de montan, de lignite, de pétrolatum, de vaseline ou les cires microcristallines, la cérésine, l'ozokérite ; les cires synthétiques comme les cires de polyéthylène, les cires obtenues par synthèse de Fischer-Tropsch et les esters linéaires résultant de la réaction d'un acide carboxylique saturé en $C_{10}$ à $C_{40}$ et d'un alcool saturé en $C_{10}$ à $C_{40}$ comme le myristate de myristyle. On peut aussi utiliser l'alcool cétylique, l'alcool stéarylique, les lanolates ou stéarates de calcium, l'huile de ricin, de palme, de coco, de tournesol ou de coprah hydrogénée.

La phase grasse de l'émulsion E/H selon l'invention peut comprendre une ou des huile(s) hydrocarbonée(s) dans une proportion allant jusqu'à 40 % en poids par rapport au poids total de la phase grasse de l'émulsion.

Comme huile hydrocarbonée, on citera : toute huile (ou mélange d'huiles) fluide stable à la température d'utilisation habituelle des produits cosmétiques, pharmaceutiques, hygiéniques telles que les huiles d'origine végétale ou animale, minérale ou synthétique, les huiles fluorées et les triglycérides d'acides gras en $C_{12}$-$C_{18}$.

Parmi les huiles d'origine végétale ou animale, modifiées ou non, on peut citer par exemple l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau (d'abricot), l'huile de calophyllum.

Parmi les huiles d'origine minérale, on peut citer par exemple l'huile de paraffine.

Parmi les huiles synthétiques, on peut citer notamment les isoparaffines volatiles ou non et les poly-isobutènes.

Ces corps gras peuvent en particulier être choisis de manière variée par l'homme du métier afin de préparer une composition ayant les propriétés souhaitées, par exemple en consistance ou en texture. Ils sont de préférence utilisés à une teneur inférieure ou égale à 7 % en poids par rapport au poids total de l'émulsion, afin de conserver les propriétés avantageuses de l'émulsion utilisée selon l'invention.

Parmi les autres adjuvants liposolubles que l'on peut incorporer à la phase grasse, on peut citer les filtres U.V. lipophiles, les vitamines lipophiles, les antioxydants et les parfums, les céramides.

La phase aqueuse de l'émulsion selon l'invention peut comprendre de l'eau ou une eau florale telle que l'eau de bleuet.

En outre, la phase aqueuse peut comprendre de 0 % à 14 % en poids, par rapport au poids total de la phase aqueuse, d'un monoalcool inférieur en $C_2$-$C_6$ et/ou d'un polyol tel que le glycérol, le butylèneglycol, l'isoprèneglycol, le propylèneglycol.

La phase aqueuse peut également renfermer des adjuvants communément utilisés dans les émulsions E/H cosmétiques. On citera par exemple les lubrifiants, les agents hydratants, tels que le glycérol et le propylène glycol, les oligo-éléments, les filtres UV hydrophiles, les polysaccharides ainsi que des électrolytes tels que NaCl ou MgSO4. Elle peut également comprendre des principes actifs tels que des extraits végétaux, des extraits bactériens, des protéines ou leurs hydrolysats et notamment des hydrolysats de collagène ou d'élastine.

Ces principes actifs peuvent être présents en une proportion comprise entre 1 et 15 %.

Selon la texture désirée pour l'émulsion selon l'invention, la proportion de la phase aqueuse dispersée peut aller de 35 % à 80 %.

D'une manière générale, l'émulsion selon l'invention peut comprendre de 30 % à 55 % en poids de phase grasse, de 5 % à 12 % en poids de tensioactif, et de 35 % à 75 % en poids de phase aqueuse.

En outre, l'émulsion selon l'invention peut comprendre un ou plusieurs cotensioactifs, un ou plusieurs agents épaississants dans des concentrations préférentielles allant de 0 à 6 % en poids, par rapport au poids total de l'émulsion.

L'agent épaississant peut être choisi parmi les argiles modifiées telles que le silicate de magnésium modifié (bentone gel VS38 de RHEOX), l'hectorite modifiée par le chlorure de distéaryl diméthyl ammonium (bentone 38 CE de RHEOX).

De préférence, l'émulsion selon l'invention est substantiellement exempte d'agent épaississant.

L'émulsion selon l'invention comprend une phase particulaire comprenant des pigments et/ou des nacres et/ou des charges habituellement utilisés dans les compositions cosmétiques.

Les pigments peuvent être présents dans l'émulsion à raison de 0-20 % en poids, par rapport au poids total de l'émulsion, et de préférence à raison de 2-15 %. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique, les nacres telles que le mica recouvert d'oxyde de titane, d'oxyde de fer, de pigment naturel ou d'oxychlorure de bismuth ainsi que le mica titane coloré. Parmi les pigments organiques, on peut citer le noir de carbone, et les laques de baryum, strontium, calcium, aluminium. Les pigments peuvent également présenter une surface hydrophobe ou peuvent être traités de manière à rendre leur surface hydrophobe ; ce traitement peut être effectué selon les méthodes connues de l'homme du métier ; les pigments peuvent notamment être enrobés par des composés siliconés tels que des PDMS et/ou par des polymères, notamment des polyéthylènes et/ou des acides aminés. Les pigments enrobés peuvent également être traités afin de rendre leur surface hydrophile.

Parmi les pigments enrobés, on peut citer notamment les pigments vendus sous la dénomination de "Covasil"

par la Société WACKER (pigments au triisostéaroyltitanate).

Les pigments ainsi enrobés peuvent être incorporés dans l'émulsion selon l'invention en une proportion allant de 0,1 à 15 % en poids par rapport au poids total de l'émulsion.

Les charges, qui peuvent être présentes dans l'émulsion à raison de 0-20 % en poids, par rapport au poids total de l'émulsion, de préférence 0-10 %, peuvent être minérales ou de synthèse, lamellaires ou non lamellaires. On peut citer le talc, le mica, la silice, le kaolin, le Téflon, l'amidon, la nacre naturelle, le nitrure de bore, les microsphères telles que l'Expancel (Nobel Industrie), les microéponges telles que le polytrap (Dow Corning). De préférence, on utilise des charges sphériques dont la taille est inférieure à 25 µm telles que les poudres de polyéthylène, les poudres de Nylon, les microbilles de résine de silicone (Tospearls de Toshiba), les microsphères de silice, ces charges pouvant contribuer à améliorer les propriétés non transfert des émulsions de l'invention.

Dans une forme préférée de réalisation de l'invention, l'émulsion comprend des charges dont la taille moyenne des particules est inférieure ou égale à 15 microns. De préférence encore, ces charges sont non sphériques. De préférence, le rapport pondéral des charges sur les huiles dans la composition, après application sur la peau et après évaporation des huiles volatiles, va de 30:70 à 50:50. De préférence encore, si on désigne par $n_1$ l'indice de réfraction moyen de l'ensemble des charges et par $n_2$ l'indice de réfraction moyen de l'ensemble des huiles, alors on a :

$$0 < |n_1 - n_2| \leq 0,3$$

et de préférence,

$$0 < |n_1 - n_2| \leq 0,15,$$

On peut ainsi obtenir un fond de teint qui comprend peu de pigments mais qui camoufle bien les micro-reliefs de la peau. Cette composition présente des propriétés de soft-focus, autrement dit, lorsqu'on l'applique sur la peau, elle donne un effet flou qui camoufle les micro-reliefs de la peau.

L'émulsion selon l'invention peut également comprendre un composé filmogène.

Ainsi, l'émulsion selon l'invention peut comprendre des polymères en dispersion aqueuse, comme par exemple des polymères acryliques, polyesters et/ou polyuréthannes en dispersion aqueuse. Par exemple, la composition peut comprendre un copolymère acétate de vinyle/p-tertio-butyl-benzoate de vinyl/acide crotonique en dispersion aqueuse partiellement neutralisé, stabilisé.

L'émulsion selon l'invention peut également comprendre une dispersion de particules de polymère dans un milieu non aqueux, comme décrit par exemple dans le document EP 749747.

L'émulsion selon l'invention peut comprendre en outre un milieu cosmétiquement, pharmaceutiquement ou hygiéniquement acceptable. Elle peut comprendre alors tout additif usuellement utilisé dans le domaine cosmétique, pharmaceutique ou hygiénique, tels que des antioxydants, des colorants, des parfums, des huiles essentielles, des conservateurs, des actifs cosmétiques, des hydratants, des vitamines, des sphingolipides, des polymères liposolubles notamment hydrocarbonés, tels que le polybutène, les polyalkylènes, les polyacrylates et les polymères siliconés compatibles avec les corps gras.

Ces additifs peuvent être présents dans la composition à raison de 0-10% en poids.

Bien entendu l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Les émulsions selon l'invention se présentent sous la forme d'un produit cosmétique et notamment sous la forme d'un produit de maquillage, en particulier un fond de teint, un fard à joues ou à paupières, un eye-liner, un mascara ou un rouge à lèvres.

Elles peuvent également se présenter sous forme non colorée, contenant éventuellement des actifs cosmétiques. L'émulsion selon l'invention peut être sous forme d'une émulsion épaissie, d'une émulsion fluide, d'une crème, d'un lait ou d'un sérum, susceptible d'être utilisé comme produit de soin ou solaire.

Les émulsions selon l'invention ont une viscosité dynamique allant de 100 mPa.s à 20 Pa.s (100 cps à 200 poises), cette viscosité étant mesurée à 25 °C à un taux de cisaillement de 200 $s^{-1}$.

Par exemple, cette viscosité peut être mesurée sur un Rhéomat 180 de METTLER en utilisant le module 2.

De préférence, cette viscosité va de 150 mPa.s à 5 Pa.s (150 cps à 50 poises), cette viscosité étant mesurée à 25 °C à un taux de cisaillement de 200 $s^{-1}$.

Les compositions selon l'invention présentent l'avantage d'être à la fois fluides et particulièrement stables.

Le procédé de préparation des émulsions selon l'invention consiste : (a) en un premier temps, à chauffer la phase grasse contenant le système émulsionnant jusqu'à une température suffisante pour fondre tous les constituants, de préférence entre 60 et 85 °C puis à y incorporer les adjuvants liposolubles additionnels éventuels, et (b) en un deuxième temps, après refroidissement de la phase grasse entre 40 et 60 °C, à ajouter la phase aqueuse, portée à la même température, à la phase grasse sous faible agitation et de manière lente, puis lorsque la température est revenue à environ 25 °C, à soumettre alors la préparation à une forte agitation.

Cette deuxième étape peut être également réalisée par addition sous vive agitation de la phase aqueuse à la phase grasse, la phase aqueuse étant portée à la même température que la phase grasse.

On va maintenant donner à titre d'illustration, plusieurs exemples de compositions cosmétiques sous forme d'émulsions E/H. Dans les exemples qui suivent, les quantités sont données en pourcentage en poids par rapport au poids total de la composition.

**EXEMPLE COMPARATIF :**

La Demanderesse a réalisé les deux émulsions A et B suivantes de viscosités dynamiques similaires (- 500 mPa.s (500 cps) à T° = 25 °C). Ces viscosités ont été mesurées avec un viscosimètre RM180 Rhéomat (Rheometric Scientific) de Mobile 2, à la température de 25 °C et au taux de cisaillement $200s^{-1}$, au temps t = 10 minutes.

L'émulsion A comprend une silicone oxyalkylénée en $\alpha$-$\omega$ conforme à l'invention. L'émulsion B comprend une silicone oxyalkylénée dont les groupes oxyalkylénés ne sont pas aux extrémités de la chaîne siliconée mais pendants à cette chaîne. L'émulsion B comprend également des agents épaississants (diphényl dimethicone et hectorite) pour obtenir la viscosité désirée.

Emulsion A (conforme à l'invention) :

- mélange silicone oxyéthylénée oxypropylénée substituée en $\alpha$-$\omega$ /cyclométhicone (85/15) vendu sous la dénomination commerciale "Abil EM 97" par la Société Goldschmidt 6 %
- isostéaryl diglycéryl succinate vendu sous la dénomination commerciale "Imwitor 780 K" par la Société Hüls 2 %
- cyclométhicone 25 %
- isododécane 4,55 %
- pigment 10 %
- poudre de Nylon 8 %
- copolymère acétate de vinyle/p-tertio-butyl-benzoate de vinyl/acide crotonique en dispersion aqueuse partiellement neutralisé, stabilisé 20 %
- adipate de diisopropyle 1 %
- eau qs 100 %

Emulsion B (comparative) :

- silicone à groupements alkyle, oxyéthylène et oxypropylène pendants dans un mélange polyglycéryl-4-isostéarate et hexyl laurate vendue sous la dénomination commerciale "Abil WE 09" par la Société Goldschmidt 9 %
- mélange stéarate de glycol acétylé et tristéarine vendu sous la dénomination commerciale "Unitwix" par la Société Guardian 0,5 %
- cyclométhicone 25 %
- diphényl diméthicone 6 %
- isododécane 4,55 %
- hectorite 4 %
- pigment 10 %
- poudre de Nylon 8 %
- copolymère acétate de vinyle/p-tertio-butyl-benzoate de vinyl/acide crotonique en dispersion aqueuse partiellement neutralisé, stabilisé 20 %
- adipate de diisopropyle 1 %
- eau qs 100 %

Ces émulsions ont été obtenues selon le procédé de préparation suivant :

- on prédisperse les pigments dans une partie de la cyclométhicone.

- On homogénéise le reste d'huile avec les tensioactifs (à 40-50 °C pour l'émulsion B, à froid pour l'émulsion A).

- On laisse refroidir. On a ajouté les pigments dans ce mélange, la poudre de nylon, (et l'hectorite modifiée prégonglée dans un peu d'isododécane, pour l'émulsion B).

- On a ajouté l'ensemble de la phase aqueuse dans la phase grasse précédente, sous agitation lente d'abord, puis à forte puissance pendant 10 minutes.

- On a ajouté, sous agitation lente, le copolymère et l'adipate de diisopropyle.

Ces deux fonds de teint ont une texture crémeuse, légère et douce. Leur application sur la peau est facile et donne à la peau un aspect velouté. Le toucher est très doux. Le confort est bon.

<u>1°) Evaluation de la stabilité :</u>

L'émulsion A selon l'invention présente une meilleure stabilité après 1 mois à T° = 45 °C que l'émulsion B (pour l'émulsion B, on observe un fort relarguage d'huile en surface de l'émulsion ainsi que de nombreux signes de lâchage de l'émulsion et de réagglomération des pigments au microscope) et ce, même en l'absence de composés stabilisants tels que la gomme de silicone (diphényl diméthicone) et l'hectorite qui sont présents dans l'émulsion B.

<u>2°) Evaluation de la résistance au transfert :</u>

La Demanderesse a évalué la résistance au transfert de ces deux compositions de la manière suivante : les émulsions A et B ont été appliquées comparativement par demi-cou sur un panel de 6 personnes. Puis on a laissé sécher les produits pendant 10 minutes. Ont ensuite été appliquées des collerettes pendant 30 minutes sur chaque demi-cou.

Les résultats obtenus sont rassemblés dans le tableau suivant:

| | Transfert | Emulsion A (invention) | Emulsion B (comparative) |
|---|---|---|---|
| Nul | 0 | | |
| Traces | 1 | | |
| Traces + | 2 | | |
| Léger | 3 | 3 | |
| Léger + | 4 | 1 | 1 |
| Moyen | 5 | 2 | 4 |
| Moyen + | 6 | | 1 |
| Important | 7 | | |
| **SCORE** | **(moyenne)** | **3,8** | **5** |

Il ressort clairement du tableau ci-dessus que pour une viscosité similaire la composition selon l'invention qui comprend la silicone oxyalkylénée particulière de l'invention transfère moins qu'une émulsion de l'état de la technique comprenant une silicone oxyalkylénée non conforme à celle de l'invention et des agents épaississants. Ainsi, l'émulsion selon l'invention permet d'obtenir une composition cosmétique fluide, stable et qui transfère peu, même sans la présence d'agents épaississants comme la diphényl diméthicone ou l'hectorite.

**<u>EXEMPLE DE COMPOSITION :</u>**

La Demanderesse a réalisé la composition suivante :

- pigments enrobés lipophiles 3 %

- talc de taille moyenne des particules inférieure à 15 microns 8 %
- sulfate de Mg 0,7 %
- mélange silicone oxyéthylénée oxypropylénée substituée en α-ω /cyclométhicone (85/15) vendu sous la dénomination commerciale "Abil EM 97" par la Société Goldschmidt 6 %
- isostéaryl diglycéryl succinate vendu sous la dénomination commerciale "Imwitor 780 K" par la Société Hüls 2 %
- cyclométhicone 25 %
- polydiméthysiloxane 4 %
- isododécane 4,5 %
- conservateurs qs
- eau qsp 100%

Cette composition, bien que comprenant peu de pigments, camoufle avantageusement les micro-reliefs de la peau.

## Revendications

1. Utilisation d'une émulsion eau-dans-huile de maquillage, comprenant une phase aqueuse et une phase grasse comprenant une huile de silicone, **caractérisée par le fait qu'**elle comprend une phase particulaire comprenant des pigments et/ou des nacres et/ou des charges, et au moins une silicone oxyalkylénée substituée en α-ω aux deux extrémités de la chaîne principale, la composition ayant une viscosité dynamique allant de 100 mPa.s à 20 Pa.s, cette viscosité étant mesurée à 25 °C à un taux de cisaillement de 200 $s^{-1}$ pour obtenir un maquillage qui ne transfère pas après son application sur la peau

2. Utilisation selon la revendication 1, **caractérisée par le fait que** l'émulsion a une viscosité dynamique allant de 150 mPa.s à 5 Pa.s, cette viscosité étant mesurée à 25 °C à un taux de cisaillement de 200 $s^{-1}$.

3. Utilisation selon l'une quelconque des revendications 1 et 2, **caractérisée par le fait que** la silicone oxyalkylénée répond à la formule (I) suivante :

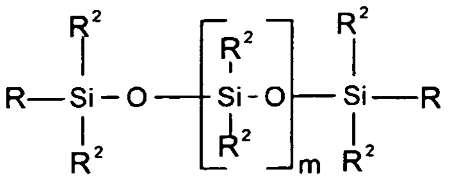

(I)

dans laquelle : R = $-(CH_2)_p$ O-(C2H4O)x $(C_3H_6O)_yR^1$
où :- $R^1$ représente H, $CH_3$ ou $CH_2CH_3$,

- p est un entier allant de 1 à 5, x varie de 1 à 100, y varie de 0 à 50,
- les unités $(C_2H_4O)$ et $(C_3H_6O)$ pouvant être réparties de façon aléatoire

ou par blocs,

- les radicaux $R^2$ représentent un radical alkyle en C1-C3 ou un radical phényle,
- $5 \leq m \leq 300$.

**4.** Utilisation selon la revendication 3, **caractérisée par le fait que** tous les radicaux $R^2$ sont des radicaux méthyles et :

- p va de 2 à 4,
- x va de 3 à 100,
- m va de 50 à 200.

**5.** Utilisation selon l'une quelconque des revendications 3 ou 4, **caractérisée par le fait que** le poids moléculaire moyen de R va de 800 à 2600.

**6.** Utilisation selon l'une quelconque des revendications 3 à 5, **caractérisée par le fait que** le rapport en poids des unités $C_2H_4O$ par rapport aux unités $C_3H_6O$ va de 100:10 à 20:80.

**7.** Utilisation selon la revendication 6, **caractérisée par le fait que** ce rapport est d'environ 42/58.

**8.** Utilisation selon l'une quelconque des revendications 3 à 7, **caractérisée par le fait que** $R^1$ est le groupe méthyle.

**9.** Utilisation selon l'une quelconque des revendications précédentes,
**caractérisée par le fait que** la silicone oxyalkylénée répond à la formule suivante :

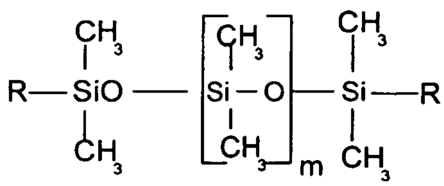

dans laquelle :

- m = 100,
- R = $(CH_2)_3$-O-$(C_2H_4O)_x$-$(C_3H_6O)_y$-$CH_3$, où x va de 3 à 100, y va de 1 à 50, le rapport en poids du nombre de $C_2H_4O$ sur le nombre de $C_3H_6O$ étant d'environ 42/58, le poids moléculaire moyen de R allant de 800 à 1000.

**10.** Utilisation selon l'une quelconque des revendications précédentes, **caractérisée par le fait que** la silicone est présente dans la composition en une proportion allant de 0,1 à 30 %, de préférence de 0,5 à 10 %, en poids par rapport au poids total de l'émulsion.

**11.** Utilisation selon l'une quelconque des revendications précédentes, **caractérisée par le fait que** l'huile de silicone est choisie parmi les polydiorganosiloxanes linéaires, éventuellement fonctionnalisés, ou cycliques ou les organopolysiloxanes éventuellement réticulés ou un mélange de ceux-ci.

**12.** Utilisation selon l'une quelconque des revendications précédentes, **caractérisée par le fait que** l'huile de silicone est un polydiorganosiloxane cyclique.

**13.** Utilisation selon l'une quelconque des revendications 1 à 12, **caractérisée par le fait que** l'huile de silicone est présente en une proportion d'au moins 5 % et de préférence allant de 25 à 45 % en poids par rapport au poids total de l'émulsion.

**14.** Utilisation selon l'une quelconque des revendications 1 à 13, **caractérisée par le fait que** l'émulsion comprend en outre au moins un corps gras non siliconé choisi parmi des corps gras pâteux, des gommes, des cires et des huiles d'origine végétale, minérale, animale ou synthétique.

**15.** Utilisation selon l'une quelconque des revendications précédentes, **caractérisée par le fait que** la charge choisie parmi le talc, le mica, la silice, le kaolin, le Téflon, l'amidon, la nacre naturelle, le nitrure de bore, les microsphères, les microéponges, les poudres de polyéthylène, les poudres de Nylon, les microbilles de résine de silicone, les microsphères de silice.

16. Utilisation selon l'une quelconque des revendications précédentes, **caractérisée par le fait que** les charges ont une taille moyenne des particules est inférieure ou égale à 15 microns.

17. Utilisation selon l'une quelconque des revendications précédentes, **caractérisée par le fait que** les charges ne sont pas sphériques.

18. Utilisation selon l'une quelconque des revendications précédentes, **caractérisée par le fait que** le rapport pondéral des charges sur les huiles dans la composition, après application sur la peau et après évaporation des huiles volatiles, va de 30:70 à 50:50.

19. Utilisation selon l'une quelconque des revendications précédentes, **caractérisée par le fait que**, si $n_1$ désigne l'indice de réfraction moyen de l'ensemble des charges et $n_2$ désigne l'indice de réfraction moyen de l'ensemble des huiles, alors :

$$0 < |n_1 - n_2| \leq 0,3$$

et de préférence,

$$0 < |n_1 - n_2| \leq 0,15.$$

20. Utilisation selon l'une quelconque des revendications précédentes, **caractérisée par le fait que** l'émulsion comprend en outre un composé filmogène.

21. Utilisation selon l'une quelconque des revendications précédentes, **caractérisée par le fait que** l'émulsion est substantiellement exempte d'agent épaississant.

22. Utilisation selon l'une quelconque des revendications précédentes, **caractérisée par le fait que** l'émulsion est substantiellement exempte de gomme de silicone.

23. Utilisation selon l'une quelconque des revendications précédentes, **caractérisée par le fait que** l'émulsion est sous la forme d'un fond de teint, d'un fard à joues ou à paupières, d'un eye-liner, d'un mascara ou d'un rouge à lèvres.

24. Utilisation selon l'une quelconque des revendications précédentes, **caractérisée par le fait que** l'émulsion est sous forme d'une émulsion épaissie, ou d'une émulsion fluide.

## Claims

1. Use of a water-in-oil makeup emulsion, comprising an aqueous phase and a fatty phase comprising a silicone oil, **characterized in that** it comprises a particulate phase comprising pigments and/or nacres and/or fillers, and at least one oxyalkylenated silicone $\alpha,\omega$-substituted at the two ends of the main chain, the composition having a dynamic viscosity ranging from 100 mPa.s to 20 Pa.s, this viscosity being measured at 25°C at a shear rate of 200 $s^{-1}$, to obtain a makeup that does not transfer after it has been applied to the skin.

2. Use according to Claim 1, **characterized in that** the emulsion has a dynamic viscosity ranging from 150 mPa.s to 5 Pa.s, this viscosity being measured at 25°C at a shear rate of 200 $s^{-1}$.

3. Use according to either of Claims 1 and 2, **characterized in that** the oxyalkylenated silicone corresponds to formula (I) below:

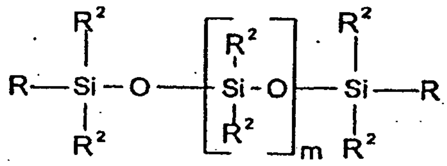

(I)

in which: R = $-(CH_2)_p-O-(C_2H_4O)_x(C_3H_6O)_yR^1$
in which: - $R^1$ represents H, $CH_3$ or $CH_2CH_3$,

- p is an integer ranging from 1 to 5, x ranges from 1 to 100, and y ranges from 0 to 50,
- the units $(C_2H_4O)$ and $(C_3H_6O)$ possibly being distributed randomly or in blocks,
- the radicals $R^2$ represent a $C_1$-$C_3$ alkyl radical or a phenyl radical,
- $5 \leq m \leq 300$.

**4.** Use according to Claim 3, **characterized in that** all the radicals $R^2$ are methyl radicals, and:

- p ranges from 2 to 4,
- x ranges from 3 to 100,
- m ranges from 50 to 200.

**5.** Use according to either of Claims 3 and 4, **characterized in that** the average molecular weight of R ranges from 800 to 2600.

**6.** Use according to any one of Claims 3 to 5, **characterized in that** the weight ratio of the units $C_2H_4O$ relative to the units $C_3H_6O$ ranges from 100:10 to 20:80.

**7.** Use according to Claim 6, **characterized in that** this ratio is about 42/58.

**8.** Use according to any one of Claims 3 to 7, **characterized in that** $R^1$ is a methyl group.

**9.** Use according to any one of the preceding claims, **characterized in that** the oxyalkylenated silicone corresponds to the following formula:

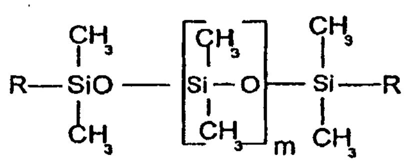

in which:

- m = 100,
- R = $(CH_2)_3-O-(C_2H_4O)_x-(C_3H_6O)_y-CH_3$, in which x ranges from 3 to 100, y ranges from 1 to 50, the weight ratio of the number of $C_2H_4O$ to the number of $C_3H_6O$ being about 42/58, and the average molecular weight of R ranging from 800 to 1000.

**10.** Use according to any one of the preceding claims, **characterized in that** the silicone is present in the composition in a proportion ranging from 0.1% to 30% and preferably from 0.5% to 10% by weight relative to the total weight of the emulsion.

**11.** Use according to any one of the preceding claims, **characterized in that** the silicone oil is chosen from optionally functionalized linear polydiorganosiloxanes, or cyclic polydiorganosiloxanes, or optionally crosslinked organopolysiloxanes, or a mixture thereof.

**12.** Use according to any one of the preceding claims, **characterized in that** the silicone oil is a cyclic polydiorganosiloxane.

**13.** Use according to any one of Claims 1 to 12, **characterized in that** the silicone oil is present in a proportion of at least 5% and preferably ranging from 25% to 45% by weight relative to the total weight of the emulsion.

**14.** Use according to any one of Claims 1 to 13, **characterized in that** the emulsion also comprises at least one non-silicone fatty substance chosen from pasty fatty substances, gums, waxes and oils of plant, mineral, animal or synthetic origin.

**15.** Use according to any one of the preceding claims, **characterized in that** the filler is chosen from talc, mica, silica, kaolin; Teflon, starch, natural mother-of-pearl, boron nitride, microspheres, microsponges, polyethylene powders, Nylon powders, silicone resin microbeads and silica microspheres.

**16.** Use according to any one of the preceding claims, **characterized in that** the fillers have a mean particle size of less than or equal to 15 microns.

**17.** Use according to any one of the preceding claims, **characterized in that** the fillers are not spherical.

**18.** Use according to any one of the preceding claims, **characterized in that** the weight ratio of the fillers to the oils in the composition, after application to the skin and after evaporation of the volatile oils, ranges from 30:70 to 50:50.

**19.** Use according to any one of the preceding claims, **characterized in that**, if $n_1$ denotes the mean refractive index of the fillers as a whole and $n_2$ denotes the mean refractive index of the oils as a whole, then:

$$0 < | n_1 - n_2 | \leq 0.3$$

and preferably

$$0 < | n_1 - n_2 | \leq 0.15.$$

**20.** Use according to any one of the preceding claims, **characterized in that** the emulsion also comprises a film-forming compound.

**21.** Use according to any one of the preceding claims, **characterized in that** the emulsion is substantially free of thickener.

**22.** Use according to any one of the preceding claims, **characterized in that** the emulsion is substantially free of silicone gum.

**23.** Use according to any one of the preceding claims, **characterized in that** the emulsion is in the form of a foundation, a makeup rouge, an eyeshadow, an eyeliner, a mascara or a lipstick.

**24.** Use according to any one of the preceding claims, **characterized in that** the emulsion is in the form of a thickened emulsion, or a fluid emulsion.

## Patentansprüche

**1.** Verwendung einer Wasser-in-Öl-Emulsion zum Schminken, die eine wässrige Phase und eine Fettphase mit einem Siliconöl enthält, **dadurch gekennzeichnet, dass** sie eine Partikelphase, die Pigmente und/oder Perlglanzpigmente und/oder Füllstoffe enthält, und mindestens ein in $\alpha$-$\omega$-Stellung an beiden Seiten der Hauptkette substituiertes alkoxyliertes Silicon enthält, wobei die Zusammensetzung eine dynamische Viskosität von 100 mPa·s bis 20 Pa·s aufweist, wobei die Viskosität bei einem Schergrad von 200 $s^{-1}$ bei 25 °C bestimmt wird, um eine Schminke zu bilden, die sich nach dem Aufbringen auf die Haut nicht überträgt.

2. Verwendung nach Anspruch 1, **dadurch gekennzeichnet, dass** die Emulsion eine dynamische Viskosität von 150 mPa·s bis 5 Pa·s aufweist, wobei die Viskosität bei einem Schergrad von 200 $s^{-1}$ bei 25 °C bestimmt wird.

3. Verwendung nach einem der Ansprüche 1 und 2, **dadurch gekennzeichnet, dass** das alkoxylierte Silicon der folgenden Formel I entspricht:

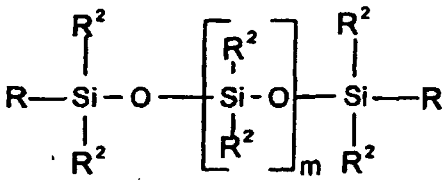

(I)

in der :

- R = $-(CH_2)_p O-(C_2H_4O)_x (C_3H_6O)_y R^1$,

worin: - $R^1$ H, $CH_3$ oder $CH_2CH_3$ bedeutet,

- p eine ganze Zahl von 1 bis 5 ist, x im Bereich von 1 bis 100 liegt und y im Bereich von 0 bis 50 liegt,
- wobei die Einheiten $(C_2H_4O)$ und $(C_3H_6O)$ zufällig oder in Form von Blöcken verteilt sein können,
- die Gruppen $R^2$ eine $C_{1-3}$-Akylgruppe oder eine Phenylgruppe bedeuten,
- $5 \leq m \leq 300$.

4. Verwendung nach Anspruch 3, **dadurch gekennzeichnet, dass** es sich bei allen Gruppen $R^2$ um Methylgruppen handelt und:

- p im Bereich von 2 bis 4 liegt,
- x im Bereich von 3 bis 100 liegt,
- m im Bereich von 50 bis 200 liegt.

5. Verwendung nach einem der Ansprüche 3 oder 4, **dadurch gekennzeichnet, dass** das mittlere Molekulargewicht von R im Bereich von 800 bis 2600 liegt.

6. Verwendung nach einem der Ansprüche 3 bis 5, **dadurch gekennzeichnet, dass** das Gewichtsverhältnis der Einheiten $C_2H_4O$ und der Einheiten $C_3H_6O$ im Bereich von 100:10 bis 20:80 liegt.

7. Verwendung nach Anspruch 6, **dadurch gekennzeichnet, dass** dieses Verhältnis etwa 42/58 beträgt.

8. Verwendung nach einem der Ansprüche 3 bis 7, **dadurch gekennzeichnet, dass** $R_1$ Methyl bedeutet.

9. Verwendung nach einem der vorhergehenden Ansprüche, **dadurch gekennzeichnet, dass** das alkoxylierte Silicon der folgenden Formel entspricht:

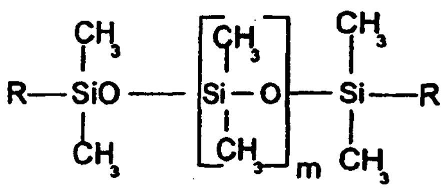

in der :

- m = 100,

- R = $(CH_2)_3$-O-$(C_2H_4O)_x$-$(C_3H_6O)_y$-$CH_3$, wobei x im Bereich von 3 bis 100 liegt und y im Bereich von 1 bis 50 liegt, wobei das Gewichtsverhältnis der Anzahl von $C_2H_4O$ und der Anzahl von $C_3H_6O$ etwa 42/58 beträgt und wobei die mittlere Molmasse von R im Bereich von 800 bis 1000 liegt.

**10.** Verwendung nach einem der vorhergehenden Ansprüche, **dadurch gekennzeichnet, dass** das Silicon in der Zusammensetzung in einer Menge von 0,1 bis 30 % und vorzugsweise 0,5 bis 10 Gew.-%, bezogen auf das Gesamtgewicht der Emulsion, enthalten ist.

**11.** Verwendung nach einem der vorhergehenden Ansprüche, **dadurch gekennzeichnet, dass** das Siliconöl unter den gegebenenfalls funktionalisierten, linearen Polydiorganosiloxanen oder cyclischen Polydiorganosiloxanen oder gegebenenfalls vernetzten Organopolysiloxanen oder deren Gemischen ausgewählt ist.

**12.** Verwendung nach einem der vorhergehenden Ansprüche, **dadurch gekennzeichnet, dass** das Siliconöl ein cyclisches Polydiorganosiloxan ist.

**13.** Verwendung nach einem der Ansprüche 1 bis 12, **dadurch gekennzeichnet, dass** das Siliconöl in einer Menge von mindestens 5 % und vorzugsweise von 25 bis 45 Gew.-%, bezogen auf das Gesamtgewicht der Emulsion, enthalten ist.

**14.** Verwendung nach einem der Ansprüche 1 bis 13, **dadurch gekennzeichnet, dass** die Emulsion ferner mindestens eine nichtsiliconhaltige Fettsubstanz enthält, die unter den pastösen Fettsubstanzen, Gummis, Wachsen und Ölen pflanzlicher, mineralischer, tierischer oder synthetischer Herkunft ausgewählt ist.

**15.** Verwendung nach einem der vorhergehenden Ansprüche, **dadurch gekennzeichnet, dass** der Füllstoff unter Talk, Glimmer, Kieselsäure, Kaolin, Teflon, Stärke, natürlichem Pigment, Bornitrid, Mikrosphären, Mikroschwämmen, Polyethylenpulvern, Nylonpulvern, Siliconharzmikrokugeln und Siliciumoxidmikrosphären ausgewählt ist.

**16.** Verwendung nach einem der vorhergehenden Ansprüche, **dadurch gekennzeichnet, dass** die Füllstoffe eine mittlere Partikelgröße von 15 µm oder darunter aufweisen.

**17.** Verwendung nach einem der vorhergehenden Ansprüche, **dadurch gekennzeichnet, dass** die Füllstoffe nichtsphärisch sind.

**18.** Verwendung nach einem der vorhergehenden Ansprüche, **dadurch gekennzeichnet, dass** das Gewichtsverhältnis der Füllstoffe und der Öle in der Zusammensetzung nach dem Aufbringen auf die Haut und nach dem Verdampfen der flüchtigen Öle im Bereich von 30:70 bis 50:50 liegt.

**19.** Verwendung nach einem der vorhergehenden Ansprüche, **dadurch gekennzeichnet, dass**, wenn $n_1$ die mittlere Brechzahl der gesamten Füllstoffe und $n_2$ die mittlere Brechzahl der gesamten Öle angibt, gilt:

$$0 < |n_1 - n_2| \leq 0{,}3$$

und vorzugsweise

$$0 < |n_1 - n_2| \leq 0{,}15.$$

**20.** Verwendung nach einem der vorhergehenden Ansprüche, **dadurch gekennzeichnet, dass** die Emulsion ferner eine filmbildende Verbindung enthält.

**21.** Verwendung nach einem der vorhergehenden Ansprüche, **dadurch gekennzeichnet, dass** die Emulsion im Wesentlichen kein Verdickungsmittel enthält.

**22.** Verwendung nach einem der vorhergehenden Ansprüche, **dadurch gekennzeichnet, dass** die Emulsion im Wesentlichen keinen Silicongummi enthält.

**23.** Verwendung nach einem der vorhergehenden Ansprüche, **dadurch gekennzeichnet, dass** die Emulsion als Makeup, Wangenrouge, Lidschatten, Eyeliner, Mascara oder Lippenstift vorliegt.

**24.** Verwendung nach einem der vorhergehenden Ansprüche, **dadurch gekennzeichnet, dass** die Emulsion als dickflüssige Emulsion oder als fluide Emulsion vorliegt.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4698178 A
- EP 331833 A
- EP 612517 A
- EP 749747 A